# EUROPEAN PATENT APPLICATION

(11) **EP 4 495 596 A1**
(43) Date of publication of application: **22.01.2025**
(21) Application number: 23382751.8
(22) Date of filing: 20.07.2023
(51) Int. Cl.: G01N 33/543

(54) **FUNCTIONALIZED NANOPARTICLES (NPS)**

(71) Applicant: Universidad de Granada, 18071 Granada (ES)
(72) Inventor: The designation of the inventor has not yet been filed
(74) Representative: Hoffmann Eitle

(57) **Abstract**

The present invention relates to the field of medicine, in particular to functionalized nanoparticles (NPs) to be used in diagnosis, to pharmaceutical compositions or nanodevices that comprise them, and also to the method for obtaining said functionalized nanoparticles.

## Description

### TECHNICAL FIELD OF THE INVENTION

The present invention relates to the field of medicine, in particular to functionalized nanoparticles (NPs) to be used in diagnosis, to pharmaceutical compositions or nanodevices that comprise them, and also to the method for obtaining said functionalized nanoparticles.

### BACKGROUND ART

In recent years, extracellular vesicles (EVs), and particularly a subpopulation called exosomes, have gained prominence in the field of biomarkers and diagnostics. All living cells secrete these membrane-derived nanoscopic vesicles; EVs are naturally occurring particles surrounded by a lipid bilayer that carry transmembrane proteins on their surface, as well as other proteins and nucleic acids in their lumen, such as DNA, mRNA, mRNA, and other small RNAs. These vesicles are key in intercellular communication, transferring complex molecular information from a cell of origin to other cells throughout the body, associated with a variety of physiological functions and pathological disease states.

Living cells secrete heterogeneous populations of EVs, including exosomes, microvesicles, and apoptotic bodies. Exosomes are the best-known type of EVs and are formed by the folding of intracellular membranes into endosomes that generate intraluminal vesicles (ILVs) and are secreted after the fusion of multivesicular bodies (MVBs) with the plasma membrane. Due to their intraluminal origin, these particles are usually between 50 and 150 nm in size. Microvesicles are formed by budding and direct fission of the plasma membrane to the outside, and usually have a size between 100-1000nm. Apoptotic bodies are formed during cell apoptosis and contain multiple cell fragments and DNA, giving them heterogeneous characteristics in terms of size, morphology, and content. Due to the cellular processes associated with the biogenesis of EVs, different content can be selectively incorporated into each type of EV. To distinguish EVs derived from plasma membrane or intracellular membrane compartments, tetraspanins such as CD63, CD81 and CD9 are frequently used. CD63 has been suggested to be a key component for EVs originating from the plasma membrane, as it is largely found on exosomes along with other tetraspanins. Therefore, tetraspanin CD63 has been used to identify exosomes in many studies.

Exosomes are secreted around the cells of origin and released into biofluids, such as blood, urine, cerebrospinal fluid, and lymph, thus reaching distant locations in the body. Since exosomes derived from many cells in the body can be found in biofluids, identification of their cell of origin, monitoring of their content and biodistribution, and determination of their fate in recipient cells remain key issues that still need to be addressed. in this field. Knowing the content of EVs and their capacity to transport biomolecules will allow exploring their potential as a biomarker to monitor the state of a disease in response to therapy, particularly in hard-to-reach places where biopsies are extremely difficult or not possible to obtain.

The fact that exosomes are very stable in body fluids makes them perfect reservoirs for disease biomarkers and, in particular, tumour exosomes released into the blood are a great diagnostic source. Cancer cell-derived exosomes that are introduced into the blood sample are stored for a long period of time, making them an excellent tool for biomarker analysis.

The isolation of exosomes or other subtypes of EVs from biofluids remains challenging due to their heterogeneity, the current lack of specific surface markers for isolation, and the understanding of their cellular origin. The current purification methods that isolate the largest number of extracellular materials are mainly polymer precipitation kits and those based on serial ultracentrifugation (UC). These approaches are not capable of differentiating specific populations of exosomes from different cell origins or other subtypes of EVs or free proteins, necessary for the study of specific tissue-derived exosome biomarkers. Indeed, bulk measurement of a mixture of vesicle populations could potentially mask essential biomarkers, severely impairing investigations of associated pathological mechanisms, making non-specific exosomes one of the main contaminants of preparations. Being a highly enriched source of biomarkers, the use of exosomes would allow the identification of specific multiomics molecular information of their pathogenesis.

There is a wide variety of commercial precipitation and affinity kits for isolating EVs from biofluids, such as polymeric precipitation, tetraspanin (TSN) affinity, or transferrin kits. Polymer-based precipitation (typically using polyethylene glycol (PEG)) is commonly used to isolate EVs by creating a hydrophobic microenvironment through the interaction of highly hydrophilic polymers and the water molecules surrounding the EVs, which precipitate them as a result of depletion. of its solubility. Some of these kits are Exo-Prep (HansaBioMed, Tallinn, Estonia), Total Exosome Isolation Reagent (ThermoFisher, Waltham, MA, USA), and ExoQuick (System Biosciences, Palo Alto, CA, USA). Despite having a high isolation yield, the samples are characterized by their low purity due to the coprecipitation of proteins, nucleic acids and lipoproteins. Contamination negatively impacts the identification of exosome-associated proteins in multiomics assays, due to non-specific interactions causing false positives or specific signal blocking in both conventional immunoassays and emerging detection platforms.

Another commonly used method is isolation based on immunoaffinity with the surface proteins of exosomes. This method offers unique advantages for the isolation of exosomes from complex fluids, in terms of higher efficiency and specificity in exosome capture, as well as higher integrity of the isolated vesicles. Unlike precipitation-based techniques, these methods are more specific in terms of exosome capture, with high efficiency in recovering highly intact exosomes from complex biofluids, while reducing costs and labour time. Isolation by immunoaffinity specifically selects for a subpopulation of vesicles that co-express one or more surface markers that reveal their cell of origin and their specific function, including diagnostic markers for disease. Among them, immunomagnetic particles are the most widely used. The exosomes bind to the antibody on the magnetic particle and are separated from the other EVs by applying an external magnetic field. Some examples are exosome isolation/detection with human CD63 (Invitrogen, Waltham, MA, USA), CD81/CD63 exosome isolation kit (Miltenyi Biotec, Bergisch Gladbach, Germany) and Exo-Flow^{™} Selective Exosome Capture (System Biosciences). However, the reproducibility between samples and biofluids is not very high, which affects the subsequent multiomics analysis and the conclusions related to the biomarker profile. This variability is due to the lack of specificity of the antibody epitope, non-specific adsorption on the magnetic particle surface, the presence of contaminants in the biofluids, and poor pre-treatment of the starting material.

To counteract these limitations, some nanomaterials have been used in exosome isolation methods, due to their unique properties and versatility. For example, nanorods, metal-organic frameworks (MOFs), and nanostructured graphene oxide (GO)/polypropylene (PDA) were used as platforms to increase the binding surface area of antibodies and thus increase the sensitivity of the assays. In other studies, they coated polystyrene particles with zwitterionic MPC polymers to reduce nonspecific protein binding. On the other hand, the ExoCAs-2 methodology allows capturing and releasing exosomes using buffers with different pH, since it is a chitosan-based material that contains magnetic particles functionalized with polycationic polymers that change with a simple pH adjustment.

### BRIEF DESCRIPTION OF THE INVENTON

The present invention refers to a nanoparticle conjugate (nanoparticle conjugate of the invention) of formula A-X-B-C, wherein:
a. A is a nanoparticle preferably an amino nanoparticle such as an amino polystyrene nanoparticle (as an amino methyl nanoparticle),
b. X is a linker that is cleaved when exposed to an ultraviolet wavelength;
c. B is one or more amino acids or analogues thereof, preferably a lysine having the N-α-amino and N-ε groups thereof optionally protected by orthogonal protecting groups such as Dde and Fmoc, preferably Fmoc-Lys (Dde); and
d. C is a bioactive molecule such as an antibody that binds specifically to a target molecule, in particular to an extracellular vesicle (preferably an exosome),
wherein each of A and X, X and B, and B and C are directly linked to each other, optionally through a linker such as a PEG (Polyethylene glycol).

An embodiment refers to the nanoparticle conjugate of the invention, wherein the nanoparticle (A) is capable of being functionalized with (a) at least one imaging agent (T), and at least one bioactive molecule (D), and said conjugate is characterized by
a. the nanoparticle A being directly linked, preferably via an amide bond, optionally through a linker, to the cleavable spacer;
b. the cleavable spacer (X) being further directly linked, preferably via an amide bond, optionally through a linker such as PEG, to one or more amino acids or analogues thereof (B); and
c. the one or more amino acids or analogues thereof (B) being further directly linked, preferably via an amide bond, optionally through a linker such as PEG, to a bioactive molecule or, also optionally through the linker such as PEG, to a suitable molecule capable of binding a bioactive molecule.

A further embodiment refers to the nanoparticle conjugate of the invention, wherein the the cleavable spacer is amino-3-2-nitrophenyl propionic acid.

A further embodiment refers to the nanoparticle conjugate of the invention, wherein the suitable molecule capable of binding a bioactive molecule is such as 3-[(2-hydroxyethyl)dithio]propanoic acid.

A further embodiment refers to the nanoparticle conjugate of the invention, wherein the bioactive molecule is an extracellular vesicle (preferably exosome) binding agent such as an antibody or fragment thereof (such as antiCD63, antiCD81 and/or antiCD9 antibody or fragment thereof).

A further embodiment refers to the nanoparticle conjugate of the invention, wherein the nanoparticle conjugate is functionalized with (a) at least one imaging agent (T), wherein the T is bonded to the N-α-amino and/or N-ε groups once deprotected.

A further embodiment refers to the nanoparticle conjugate of the invention, wherein the imaging agent (T) is a fluorophore, preferably a far-red cyanine derivative (Cy7) or a metal such as a chloride hexahydrate lanthanide salt.

The present invention further refers to a method (method of the invention) for producing nanoparticles (NP) (nanoparticle conjugate of the invention) that can be functionalised, comprising the following steps :
a. conjugating the NPs to a cleavable spacer to provide cleavable NPs;
b. conjugating the resultant cleavable NPs of step a) to one or more amino acids or analogues thereof characterized by comprising orthogonal protecting groups such as Dde and Fmoc, wherein the conjugation step b) is optionally followed or preceded by one or more PEGylation steps; and
c. conjugating the product resultant from step b), optionally followed or preceded by one or more PEGylation steps, with a chemical group capable of binding to a binding agent such as an extracellular vesicle binding agent, such as an antibody.

An embodiment refers to the method of the invention, wherein the nanoparticles are polystyrene nanoparticles (NPs), or amino NPs or preferably polystyrene amino NPs.

A further embodiment refers to the method of the invention, wherein the the cleavable spacer is amino-3-2-nitrophenyl propionic acid.

A further embodiment refers to the method of the invention, wherein the suitable molecule capable of binding a bioactive molecule is such as 3-[(2-hydroxyethyl)dithio]propanoic acid.

A further embodiment refers to the method of the invention, wherein the bioactive molecule is an extracellular vesicle (preferably exosome) binding agent such as an antibody or fragment thereof (such as antiCD63, antiCD81 and/or antiCD9 antibody or fragment thereof).

A further embodiment refers to the method of the invention, wherein the method comprises starting with the product of the method of the invention:
a. removing the orthogonal protecting group, such as Dde, for conjugation of the nanoparticle to a tracking molecule such as a fluorophore or a metal such as a chloride hexahydrate lanthanide salt; and/or
b. further conjugating the product of step a) to a binding agent such as an extracellular vesicle binding agent, such as an antibody.

The present invention further refers to a nanodevice comprising the nanoparticle conjugate of the invention as well as uses of such nanodevice for isolating extracellular vesicles, preferably exosomes.

### BRIEF DESCRIPTION OF THE FIGURES

**Figure 1****.** Exosomes biomarkers, receptors and content.
**Figure 2****.** Core of principle.
**Figure 3. a)** Histograms show hydrodynamic diameter values of developed nanodevice with and without antibody, determined by DLS. Inset image is a representative TEM image of developed nanodevice; **b)** ζ-potential values of the developed nanodevice and its controls.
**Figure 4****.** Signal of coordinated lanthanide to the developed nanodevices analyzed by **a)** mass cytometry and **b)** HRTEM-EDX.
**Figure 5****.** Immunofluorescence of CD63-Tb-NPs (**30**) (dark red) and its negative control, Tb-NPs (**29**) (pink).
**Figure 6****.** Evaluation of CD63-Tb-NPs (**30**) cargo release. Nanoparticles were incubated with a rabbit anti-mouse IgG secondary antibody conjugated with Alexa Fluor^{®} 488 solution prior to UV exposure.
**Figure 7****.** Analysis of the size of isolated exosomes by UC by NTA. Inset is a representative image of HRTEM.
**Figure 8****.** Dot plots of ¹¹⁰Cd vs ¹⁵⁹Tb signal of **a)** isolated exosomes incubated with ¹¹⁰Cd-antiCD63 (1:100), **b)** Tb-NPs (**29**) and CD63-Tb-NPs (30) in absence of exosomes, **c)** Tb-NPs (**29**) and CD63-Tb-NPs (**30**) incubated with exosomes and stained with ¹¹⁰Cd-antiCD63 (1:100), and **d)** supernatants of samples in **c)** after 5 min of UV light exposure.
**Figure 9****.** Evaluation of exosomes capture and release efficiency of CD63-Tb-NPs (**30**) analyzed by HRTEM-EDX. **a)** CD63-Tb-NPs (**30**) incubated with exosomes prior to UV cleavage, and **b)** supernatant after exosomes capture and release by the nanodevice.

### DESCRIPTION

In the present invention we have developed a nanodevice for the detection of extracellular vesicles (EVs) using monoclonal antibody specific recognition applied to flow and mass cytometry. Specifically, we have implemented a covalent conjugation approach to attach exosome-specific antibodies for CD63 and CD81 tetraspanins. This strategy allows for easy and reproducible recognition of exosomes. Previous studies have reported the use of nanoparticles for exosomes detection by immune affinity, but they required the use of high-tech equipment for their analysis^{35,37-39}.

The developed nanodevices demonstrated clear functionality and recognition of exosomes, as evidenced by flow cytometry, BCA assay, and HRTEM-EDX analysis, as other reported nanomaterials employed for EVs immune-capture, such as nanowires³⁸, metal organic frameworks (MOFs)²¹ and nanostructured graphene oxide (GO)/polydopamine (PDA)³⁹. In comparison to other methods, the nanodevices developed in **this invention** offer several advantages, such as greater versatility in terms of conjugation, they are easy to handle, and do not require any sophisticated equipment. The results of our study demonstrated the successful detection and capture of fluorophore-labelled exosomes in the media using the developed nanodevice, with particularly higher capture efficiency observed with the CD81-Cy5-NPs (**25B**)**.** However, it is important to note that a minimal level of non-specific adsorption of exosomes to the nanodevices was observed in the negative control. This is a common challenge encountered when using exosome immune-capture nanodevices and it can be attributed to various factors such as protein contaminants, non-targeted extracellular vesicles (EVs), or the inherent lipidic nature of exosomes, which can interact with the nanomaterial^{21,32,36,37,41}.

In the subsequent stage of this invention, a novel nanodevice has been developed for the direct labeling of exosomes during their isolation from biological fluids. A new approach using a UV photocleavable linker has been employed to conjugate exosome-specific antibodies (CD63 as a proof of concept) enabling efficient recognition and capture of exosomes. This unique design allows for the tagging and selective release of the captured exosomes in a straightforward and reproducible manner. To further enhance the applicability of this nanodevice in mass cytometry, a lanthanide (Tb) has been successfully conjugated for specific labeling of the captured exosomes.

The nanotechnology-based approach developed in this chapter for immune-capture and release of tagged exosomes has shown promising results. Although previous methods, such as ExoCAS-2, have employed pH-adjustable buffers and chitosan-based materials with polycationic polymer-functionalized magnetic beads for capturing and releasing exosomes⁴¹.

To the best of our knowledge, this is the first instance of directly labelling immune-captured exosomes for subsequent release and analysis using mass cytometry. While many immune-capture nanodevices rely on magnetic nanomaterials^{36,38,41}, these are not compatible with mass cytometry analysis, making our approach a novel advancement in the field providing a comprehensive dataset of information from the sample in a single step, offering a valuable tool for studying exosomes.

Further development could be achieved by utilizing other exosomes specific tetraspanins such as CD81 and CD9 and other specific antibodies to isolated tumor-derived exosomes such as TS101⁴⁷. Each of these nanodevices could achieve the labelling of different exosomes with specific metals like europium and gadolinium.

The uniqueness of this platform lies in its capability to analyze individual exosomes. As each exosome passes through the nebulizer of the mass cytometer, it is individualized, resulting in a unique mass spectrum for each exosome. Consequently, specific single exosome profiling can be achieved for each developed nanodevice.

### Method of the invention

Therefore, a first aspect of the present invention relates to a method (method of the invention) for producing nanoparticles (NP) that can be bi-functionalised, comprising the following steps:
a) conjugating the NPs to a cleavable spacer such as one or more amino-3-2-nitrophenyl propionic acids to provide cleavable NPs;
b) conjugating the resultant cleavable NPs of step a) to one or more amino acids or analogues thereof characterized by comprising orthogonal protecting groups such as Dde and Fmoc, wherein the conjugation step b) is optionally followed or preceded by one or more PEGylation steps; and
c) conjugating the product resultant from step b), optionally followed or preceded by one or more PEGylation steps, with a chemical group such as 3-[(2-hydroxyethyl)dithio]propanoic acid capable of binding to a binding agent such as an extracellular vesicle binding agent, such as an antibody.

A "cleavable spacer" or "cleavable linker" refers to a chemical compound that separates two other chemical moieties, and that may be cleaved at least at one site under exposition to a MALDI laser. Most MALDI lasers usually have an ultraviolet (UV: inferior to 500 nm) wavelength, usually between 300 and 500 nm. For instance, many UV-MALDI analyzers have a pulsed nitrogen laser with a wavelength of 337 nm. Thus, a linker that is photocleavable at the wavelength of a MALDI laser may be efficiently cleaved at least at one site under exposition to a wavelength of 250 to 500 nm, preferably a wavelength of 320 to 360 nm or 320 to 350 nm, more preferably a wavelength of 337 nm, so that the UV-MALDI laser acts both to cleave the linker and to ionize the sample. Other MALDI analyser display an infrared (IR: superior to 770 nm) laser. For instance, a Nd:YAG laser (wavelength = 1060 nm), Er:YAG laser (wavelength = 2940 nm), a mid-infrared optical parametric oscillator (OPO) (wavelength = 2940 nm) or a TEA-CO2 laser (wavelength = 10600 nm) may be used as IR-MALDI lasers. A linker that is photocleavable at the wavelength of a MALDI laser may thus be efficiently cleaved at least one site under exposition to a wavelength of 1000 to 1100 nm, preferably a wavelength of 1060 nm, or a wavelength of 2900 to 3000 nm, preferably a wavelength of 2940 nm, or a wavelength of 10500 to 10700 nm, preferably a wavelength of 10600 nm, so that respectively a Nd:YAG, a Er:YAG , or a TEA-CO2 IR-MALDI laser acts both to cleave the linker and to ionize the sample.

A linker molecule X that is photocleavable at the wavelength of a MALDI laser may comprise a moiety chosen in the group constituted of: 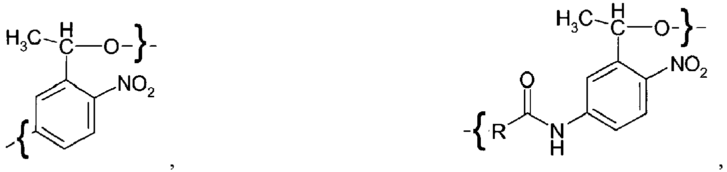 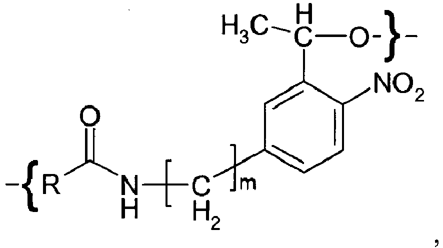 and 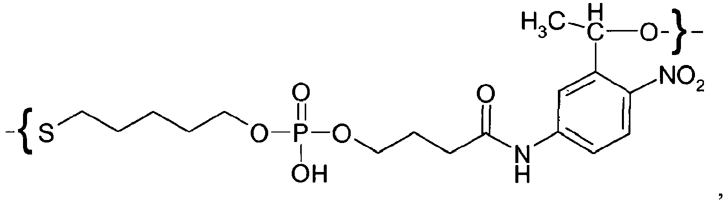 wherein R is a C1-C6 alkyl group and m is an integer comprised between 1 and 4.

A linker molecule X that is photocleavable at the wavelength of a MALDI laser may alternatively comprise a moiety chosen in the group constituted of: 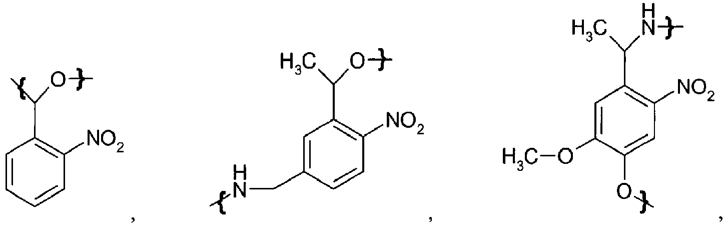 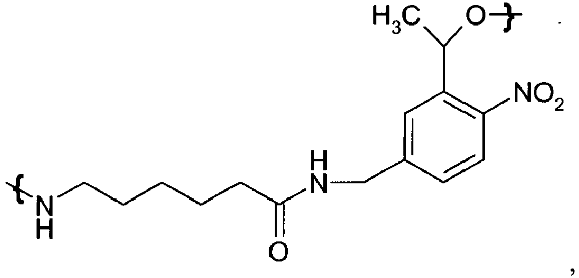 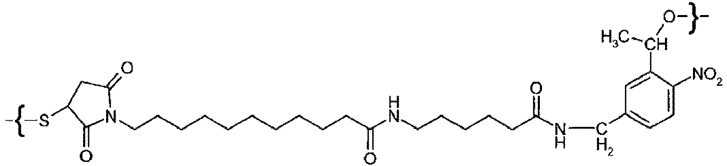 and

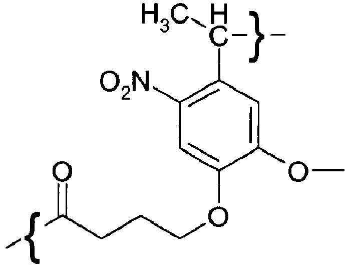

In a preferred embodiment of the first aspect of the invention, the nanoparticles are polystyrene nanoparticles (NPs), or amino NPs or preferably polystyrene amino NPs.

In another preferred embodiment of the first aspect of the invention, step a) is performed by introducing the NPs in a suitable medium, preferably dimethylformamide (DMF), in which a cleavable spacer such as amino-3-2-nitrophenyl propionic acid is preferably dissolved and activated in the medium or activated before being dissolved in the medium, for a period of time sufficient for coupling the cleavable spacer protected with Fmoc to the nanoparticles, preferably to the amino nanoparticles. Preferably, step a) is carried out by introducing the nanoparticles, preferably amino nanoparticles such as aminomethyl nanoparticles, in a suitable medium, preferably dimethylformamide (DMF), and separately a cleavable spacer such as Fmoc-amino-3-2-nitrophenyl propionic acid is dissolved in a suitable medium, preferably dimethylformamide (DMF), then a suitable reagent for amide coupling such as oxyma and/or N,N-diisopropylcarbodiimide is added to the solution comprising the cleavable spacer, then the solution is mixed with the medium comprising the nanoparticles for a period of time sufficient for coupling the cleavable spacer protected with Fmoc to the nanoparticles, preferably to the amino nanoparticles. More preferably, aminomethyl nanoparticles are suspended in N,N-dimethylformamide (DMF), separately, a cleavable spacer such as Fmoc-amino-3-2-nitrophenyl propionic acid is dissolved in DMF, then oxyma is added and the solution mixture mixed before the addition of DIC. The solution mixture is then added to the nanoparticles, preferably amino-nanoparticles, and suspension mixed to provide cleavable NPs.

In another preferred embodiment of the first aspect of the invention, step b) is performed by conjugating the resultant cleavable NPs of step a) to one or more amino acids or analogues thereof, preferably one or more lysines having the N-α-amino and N-ε groups thereof protected by orthogonal protecting groups such as Dde and Fmoc, preferably Fmoc-Lys (Dde), wherein the conjugation step b) is optionally followed or preceded by one or more PEGylation steps. Preferably, step b) is performed by conjugating the cleavable nanoparticles with one or more lysines having the N-α-amino and N-ε groups thereof protected by orthogonal protecting groups such as Dde and Fmoc, preferably Fmoc-Lys (Dde), wherein the conjugation step b) is optionally followed or preceded by one or more PEGylation steps.

It is noted that the one or more PEGylation steps can be carried out by, for example, dissolving Fmoc-PEG in a suitable medium such as DMF together with a suitable reagent for amide coupling such as oxyma and/or N,N-diisopropylcarbodiimide, and the solution mixture mixed. The solution mixture can then be added to the nanoparticles and mixed to provide the one or more PEGylation steps.

In another preferred embodiment of the first aspect of the invention, step c) can be carried out by conjugating a suitable molecule such as 3-[(2-hydroxyethyl)dithio]propanoic acid, preferably dissolved in a suitable medium such as DMF, together with a suitable reagent for amide coupling such as oxyma and/or N,N-diisopropylcarbodiimide, and the solution mixture mixed. The solution mixture is then added to nanoparticles and mixed to provide nanoparticles (NP) that can be bi-functionalised.

A non-limiting specific manner of carrying out the steps indicated in the first aspect of the invention to provide nanoparticles (NP) that can be bi-functionalised detailed herein below:
Aminomethyl nanoparticles (1 mL, 2% SC, 54 µmol/g, 1 µmοl, 1 eq) were washed in DMF (1 mL × 3 times) and suspended in N,N-dimethylformamide (DMF) (1 mL). Separately, the cleavable spacer Fmoc-amino-3-2-nitrophenyl propionic acid (15 eq) was dissolved in DMF (1 mL), then oxyma (15 eq) was added and the solution mixture mixed for 4 minutes at room temperature before the addition of DIC (15 eq) and mixed for 8-10 minutes at room temperature. The solution mixture was then added to amino-nanoparticles and suspension mixed on the Thermomixer at 1400 rpm for 2 hours at 60°C. The resulted nanoparticles were washed three times in DMF (1 mL × 3 times).
Then a capping process was achieved by the incubation of the conjugated cleavable nanoparticles with a solution of acetic anhydride/ N,N-diidopropylethylamine (50 eq) in DMF as organic solvent for 30 minutes at 1400 rpm and room temperature. The resulted nanoparticles were washed three times in DMF (1 mL × 3 times).
Fmoc deprotection was achieved by treating nanoparticles with 20% piperidine/DMF for 20 min at room temperature and 1400 rpm three times. The resulted nanoparticles were washed three times in DMF (1 mL × 3 times).
Then, Fmoc-Lys(Dde)OH (15 eq) was dissolved in DMF (1 mL), then oxyma (15 eq) was added and the solution mixture mixed for 4 minutes at room temperature before the addition of DIC (15 eq) and mixed for 8 minutes at room temperature. The solution mixture was then added to nanoparticles and suspension mixed on the Thermomixer at 1400 rpm for 2 hours at 60°C. The resulted nanoparticles were washed three times in DMF (1 mL × 3 times).
Then Fmoc deprotection step was repeated to conjugate one unit of Fmoc-PEG spacer. For that purpose, Fmoc-PEG (15 eq) was dissolved in DMF (1 mL) together with oxyma (15 eq), and the solution mixture mixed for 4 minutes at room temperature before the addition of DIC (15 eq) and mixed for 8 minutes at room temperature at 1400 rpm. The solution mixture was then added to amino-nanoparticles and suspension mixed on the Thermomixer at 1400 rpm for 2 hours at 60°C.
Then Fmoc deprotection step was repeated to conjugate 3-[(2-hydroxyethyl)dithio]propanoic acid. For that purpose, 3-[(2-hydroxyethyl)dithio]propanoic acid (10 eq) was dissolved in DMF (1 mL) together with oxyma (10 eq), and the solution mixture mixed for 4 minutes at room temperature before the addition of DIC (10 eq) and mixed for 8 minutes at room temperature at 1400 rpm. The solution mixture was then added to amino-nanoparticles and suspension mixed on the Thermomixer at 1400 rpm for 2 hours at 60°C.

On the other hand, to provide bi-functionalised nanoparticles (NP), the orthogonal protecting group, such as Dde, is removed for conjugation of the nanoparticle to a tracking molecule such as a fluorophore or a metal such as a chloride hexahydrate lanthanide salt. This lanthanide salt can be, as examples, europium(III) (Eu), gadolinium(III) (Gd), holmium(III) (Ho), palladium(II) (Pd) orterbium(III) (Tb).

Furthermore, the nanoparticle is further conjugated to a binding agent such as an extracellular vesicle binding agent, such as an antibody. For that purpose, labelled nanoparticles are conjugated to a desired binding agent such as an extracellular vesicle binding agent as an antibody (such as antiCD63, antiCD81 or antiCD9).

Therefore, a second aspect of the invention refers to the functionalization of the nanoparticles (NP) that can be bi-functionalised obtained by the method of the invention, wherein said functionalization is carried by a method comprising the following steps:
a) removing the orthogonal protecting group for conjugation of the nanoparticle to a tracking molecule such as a fluorophore or a metal such as a chloride hexahydrate lanthanide salt; and/or
b) conjugating the nanoparticle to a binding agent such as an extracellular vesicle binding agent, such as an antibody. For that purpose, nanoparticles, preferably labelled nanoparticles according to step a), are conjugated to a desired binding agent such as an extracellular vesicle binding agent as an antibody (such as antiCD63, antiCD81 or antiCD9).

Non limiting methods of providing the to provide bi-functionalised nanoparticles (NP) are detailed below:
Orthogonal protecting group Dde was removed by treating resulted nanoparticles with 2% hydrazine solution (1 mL) for 7 minutes at 1400 rpm at room temperature three times. Then nanoparticles were subsequently washed with DMF (3 × 1 m).
Then labelling of nanoparticles was carried out. Nanoparticles (1 mL, 1 eq.) were washed (2 × 1 mL) and resuspended in anhydrous DMF (1 mL). DOTA tris(acid)-amido-dPEG4 TFP ester (5 eq) and N,N-diidopropylethylamine (5 eq) was dissolved in anhydrous DMF (1 mL), and suspension mixed with nanoparticles on the Thermomixer at 1400 rpm for 15 hours at room temperature. Nanoparticles were washed then. A solution of a chloride hexahydrate lanthanide salt (1 eq) was incubated with nanoparticles for 15 hours at 1400 rpm for coordination to DOTA molecules. This lanthanide salt can be, as examples, europium(III) (Eu), gadolinium(III) (Gd), holmium(III) (Ho), palladium(II) (Pd) or terbium(III) (Tb). For Eu or Tb coordination, it requires to be dissolved in a 2:3 45 mM triethylamine (pH 9-10)/water solution at to be incubated with nanoparticles at room temperature. In the case of Gd or Ho coordination, metal must be dissolved in citrate buffer (pH 6) and incubated with nanoparticles at 50°C. After that, resulted nanoparticles were washed in deionized water (3 × 1 mL).
Finally, antibody conjugation was achieved. For that purpose, labelled nanoparticles were treated with a 1 M solution of dithiothreitol, mixing for 3 hours at 1400 rpm and room temperature in order to reduce the thiol group of the conjugated 3-[(2-hydroxyethyl)dithio]propanoic acid. Then, nanoparticles were washed with phosphate buffer saline (PBS) (3 × 1 mL).
Separately, 2.5 µM of desired antibody (such as antiCD63, antiCD81 or antiCD9) was incubated with a 50 mM solution of sulfosuccinimidyl 4-(N-maleimidomethyl)cyclohexane-1-carboxylate (sulfo-SMCC) for 30 minutes at room temperature. After this time of incubation, the excess of sulfo-SMCC was removed using centrifugal filters of 30 kDa (MWCO) by centrifugation at 14000 × g for 20 minutes, and then, in a new tube with the filter upside down, another centrifugation at 1000 × g for 2 minutes was done to obtain sulfo-functionalized purified antibodies.

In the second embodiment of the present invention, the nanoparticle is at least bifunctionalised with (a) at least one imaging agent (T), and at least one bioactive molecule (D), preferably a binding agent such as an extracellular vesicle (preferably exosome) binding agent as an antibody (such as antiCD63, antiCD81 and/or antiCD9).

In an embodiment of the present invention, the imaging agent (T) is a fluorophore, preferably a far-red cyanine derivative (Cy7) or a metal such as a chloride hexahydrate lanthanide salt.

### Nanoparticles of the invention

A third aspect of the present invention relates to a nanoparticle, preferably an amino nanoparticle such as an amino polystyrene nanoparticle (nanoparticle of the invention), capable of being functionalized with (a) at least one imaging agent (T), and at least one bioactive molecule (D), wherein said nanoparticle is characterized by
a. the nanoparticle, preferably the amino group of an amino nanoparticle such as an amino polystyrene nanoparticle (as an amino methyl nanoparticle), being directly linked, preferably via an amide bond, optionally through a linker, to a cleavable spacer such as Fmoc-amino-3-2-nitrophenyl propionic acid;
b. the cleavable spacer being further directly linked, preferably via an amide bond, optionally through a linker such as PEG, to one or more amino acids or analogues thereof, preferably a lysine having the N-α-amino and N-ε groups thereof optionally protected by orthogonal protecting groups such as Dde and Fmoc, preferably Fmoc-Lys (Dde);
c. the one or more amino acids or analogues thereof being further directly linked, preferably via an amide bond, optionally through a linker such as PEG, to a bioactive molecule or, also optionally through the linker such as PEG, to a suitable molecule such as 3-[(2-hydroxyethyl)dithio]propanoic acid capable of binding a bioactive molecule.

The nanoparticle of the invention can be thus defined as a conjugate of formula A-X-B-C, wherein:
- A is a nanoparticle preferably an amino nanoparticle such as an amino polystyrene nanoparticle (as an amino methyl nanoparticle),
- X is a linker that is cleaved according to the invention,
- B is one or more amino acids or analogues thereof, preferably a lysine having the N-α-amino and N-ε groups thereof optionally protected by orthogonal protecting groups such as Dde and Fmoc, preferably Fmoc-Lys (Dde); and
- C is an antibody that binds specifically to a target molecule, in particular to an extracellular vesicle (preferably an exosome).

Preferably, A is optionally linked to X directly or via a linker such as PEG, X is optionally linked to B directly or via a linker such as PEG, and B is optionally linked to C directly or via a linker such as PEG or via a suitable molecule such as 3-[(2-hydroxyethyl)dithio]propanoic acid capable of binding the bioactive molecule.

A non-limiting example of a conjugate of formula A-X-B-C, is:

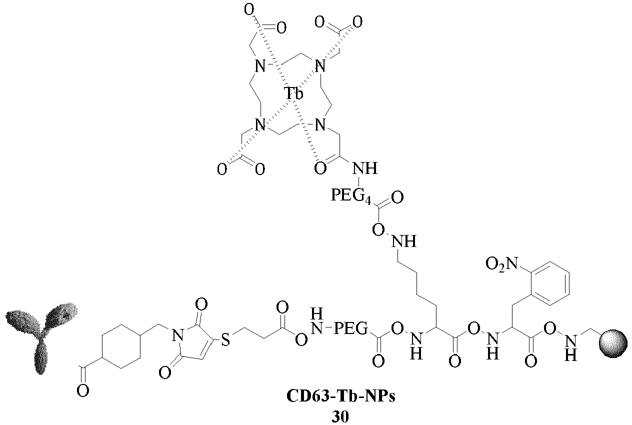

Preferably, the bioactive molecule is an extracellular vesicle (preferably exosome) binding agent as an antibody (such as antiCD63, antiCD81 and/or antiCD9).

Preferably, the nanoparticle, preferably an amino nanoparticle such as an amino polystyrene nanoparticle (nanoparticle of the invention), of the third aspect of the invention is functionalized with (a) at least one imaging agent (T), and at least one bioactive molecule (D), wherein the T is bonded to the N-α-amino and/or N-ε groups once deprotected; and wherein the D is bonded to the one or more amino acids or analogues thereof, preferably via an amide bond, optionally through a linker such as PEG, or to a suitable molecule such as 3-[(2-hydroxyethyl)dithio]propanoic acid bound, optionally through a linker, to the said one or more amino acids or analogues thereof.

In an embodiment of the present invention, the imaging agent (T) is a fluorophore, preferably a far-red cyanine derivative (Cy7) or a metal such as a chloride hexahydrate lanthanide salt. This lanthanide salt can be, as examples, europium(III) (Eu), gadolinium(III) (Gd), holmium(III) (Ho), palladium(II) (Pd) orterbium(III) (Tb).

In the third preferred embodiment of the nanoparticle of the invention, the size range of the nanoparticle is from 100 nm to 2000 nm. Preferably, the nanoparticle of the invention has a size of about 200 nm.

### Nanodevice of the invention

The fourth embodiment of the present invention relates to a nanodevice, particularly a diagnostic nanodevice, comprising any of the nanoparticles of the invention described in the third aspect of the invention or a nanoparticle obtained by means of the method of the invention.

### Uses of the nanoparticles of the invention

The fifth aspect of the present invention relates to the nanoparticle, the nanodevice, or the nanoparticle obtained by means of the method of the invention, for use in the diagnosis of cancer, or in the control of the treatment of cancer. In particular, such nanoparticle, nanodevice, or nanoparticle obtained by means of the method of the invention is used to capture extracellular vesicles such as exosomes. In particular, this can be achieved by incorporating the cleavable linker that releases the EVs such as exosomes captured due to antibody specific recognition when it is exposed to UV light. The use of mass cytometry for detecting the isolated exosomes is also encompass within the present invention.

### EXAMPLES

### MATERIALS AND METHODS

### Synthesis of CD63-Tb-NPs (30)

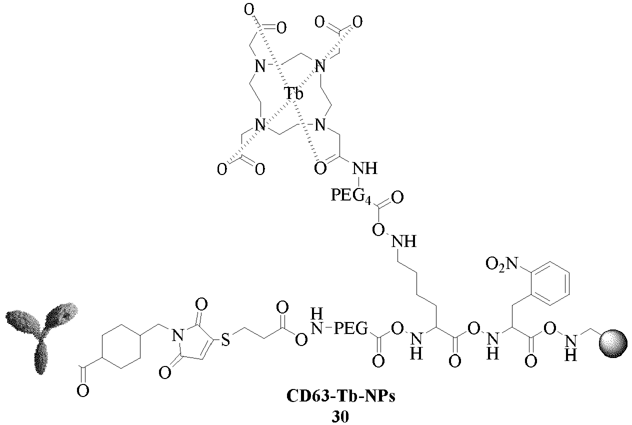

NK-NPs (**1**) (1 mL, 1 eq) were washed in *N*,*N*-dimethylformamide (DMF) (1 mL × 3 times) and suspended in DMF (1 mL). Separately, the cleavable spacer Fmoc-amino-3-2-nitrophenyl propionic acid (15 eq) was dissolved in DMF (1 mL), then oxyma (15 eq) was added and the solution mixture mixed for 4 minutes at room temperature before the addition of DIC (15 eq) and mixed for 8-10 minutes at room temperature. The solution mixture was then added to amino-NPs and suspension mixed on the Thermomixer at 1,400 rpm for 2 hours at 60°C. The resulted NPs were washed three times in DMF (1 mL × 3 times).

Then a capping process was achieved by the incubation of the conjugated cleavable NPs with a solution of acetic anhydride/ *N*,*N*-diidopropylethylamine (50 eq) in DMF as organic solvent for 30 minutes at 1,400 rpm and room temperature. The resulted NPs were washed three times in DMF (1 mL × 3 times).

Then, Fmoc deprotection was achieved as previously described prior to the conjugation of Fmoc-Lys(Dde)OH (15 eq) followed by a PEGylation step.

Then Fmoc deprotection step was repeated to conjugate 3-[(2-hydroxyethyl)dithio]propanoic acid. For that purpose, 3-[(2-hydroxyethyl)dithio]propanoic acid (10 eq) was dissolved in DMF (1 mL) together with oxyma (10 eq), and the solution mixture mixed for 4 minutes at room temperature before the addition of DIC (10 eq) and mixed for 8 minutes at room temperature at 1,400 rpm. The solution mixture was then added to amino-NPs and suspension mixed on the Thermomixer at 1,400 rpm for 2 hours at 60°C.

Orthogonal protecting group Dde was removed by treating resulted NPs with 2% hydrazine solution (1 mL).

Then, labelling of NPs was carried out. Resulting NPs (1 mL, 1 eq) were washed twice with DMF (1 mL) and resuspended in anhydrous DMF (1 mL). DOTA tris(acid)-amido-dPEG4 TFP ester (5 eq) and *N*,*N-*diidopropylethylamine (5 eq) was dissolved in anhydrous DMF (1 mL), and suspension mixed with NPs on the Thermomixer at 1,400 rpm for 15 hours at room temperature. NPs were washed and a solution of a terbium chloride hexahydrate (1 eq) was incubated with NPs for 15 hours at 1400 rpm for coordination to DOTA molecules. For that purpose, TbCl₃ · 6 H₂O requires to be dissolved in a 2:3 45 mM triethylamine (pH 9-10)/water solution. After that, resulted NPs were washed in deionized water (3 × 1 mL).

Finally, antibody conjugation was achieved by the treating them with a 1 M solution of dithiothreitol, mixing for 3 hours at 1400 rpm and room temperature in order to reduce the thiol group of the conjugated 3-[(2-hydroxyethyl)dithio]propanoic acid. Then, NPs were washed with phosphate buffer saline (PBS) (3 × 1 mL).

Separately, 2.5 µM of antiCD63 antibody was functionalized. The conjugation of reduced labelled-NPs with sulfo-antibodies was optimized incubating both of them together in PBS and suspension mixed on the Thermomixer at 1,000 rpm for 15 hours at room temperature. Finally, resulted NPs were washed with sterile PBS, and CD63-Tb-NPs (**30**) were obtained.

### UV cleavage

100 µL of sample were placed in a 96-well plate. Then, they were placed for 5 minutes in darkness on a Visi-Blue^{™} UV Transilluminator (VB-26, PIN 95-0461-02 of 220-230 V ~ 50-60 Hz and 0.2 Amps) (UVP).

### Exosomes isolation and purification

To obtain exosomes for testing the developed nanodevices, cells were cultured at standard anchorage-dependent culture conditions with fresh medium supplemented with 10% exosome-depleted FBS, until 80% confluence. FBS was depleted of bovine exosomes by ultracentrifugation (UC) at 100,000 xg for 70 min. Supernatant fractions collected from 72 h cell cultures were centrifuged at 500 xg for 10 min to remove cell debris.

Exosomes were purified by sequential centrifugation as previously described⁴⁸ with minor modifications. Briefly, to remove any possible apoptotic bodies, dead cells and large cell debris, the supernatants were first spun at 10,000 xg for 40 min at 4°C. Exosomes were collected by ultracentrifugation at 100,000 xg for 80 min at 4°C (Beckman SW28 rotor). Exosome pellets were washed with DPBS and pelleted again by ultracentrifugation at 100,000 xg for 80 min at 4°C. The final pellet was resuspended in 100 µL of DPBS and stored frozen at -80°C.

### Exosomes analysis by NTA, DLS, HRTEM and BCA

NTA analyses were performed on NanoSightNS500 instruments (Malvern Instruments, UK). The instrument was equipped with a 488 nm laser, a high sensitivity CMOS camera and a syringe pump. The measurements were analyzed using the NTA2.3 software (Malvern) after capture 3 videos of 60 sec. For DLS, HRTEM and BCA analysis, protocols were described above.

For all these methods, exosomes samples were diluted 1:1000 in DPBS buffer to obtain a concentration range (1-10 × 10⁸ particles/mL).

### Exosomes recognition by NPs by HRTEM-EDX

Ratio exosomes:NPs was the same as before. However, the samples with exosomes had a negative staining with uranyl acetate in order to be able to see them (done by the Samples Preparation Unit). It was necessary just 100 µL of exosomes-NPs solution. The HRTEM-EDX equipment used was the one mentioned in **Section** Error! Reference source not found..

### Detection of captured and released exosomes by mass cytometry

Isolated exosomes were incubated with 1 µL of CD63-Tb-NP (**30**) for 30 minutes in darkness and 25°C. To remove the non-captured exosomes, a series of centrifugations at 14,000 rpm was carried out, resuspending the pellet with DPBS. Then, samples were incubated with ¹¹⁰Cd-antiCD63 (1:100) for 30 min, and after washing steps in DPBS, they were exposed to UV light as previously described in a 96-well plate. Samples were analyzed using mass cytometry measuring ¹¹⁰Cd and ¹⁵⁹Tb intensities.

### Example 1. Development of a nanodevice for the detection of extracellular vesicles (EVs) based on monoclonal antibody specific recognition applied to flow and mass cytometry

### 1.1. Liquid Biopsy and EVs

Liquid biopsy has emerged as an alternative to conventional tissue biopsy for the diagnosis of cancer due to the (i) minimal invasiveness, (ii) higher sensitivity than standard tissue biopsies and (iii) faster sample extraction. In addition, the procedure presents minimal side effects, it is painless and has the ability to diagnose early and real time monitoring, the main disadvantages of the tissue biopsy. Liquid biopsies may include extracellular vesicles (EVs), circulating tumor cells (CTCs), and cell-free circulating tumor DNA (ctDNA)¹. This emerging technique allows the detection of biomarkers of specific pathologies with difficult access for tissue biopsy, allowing an early diagnosis and monitoring the disease progression².

Living cells secrete heterogeneous populations of membrane vesicles, including (i) exosomes, 50-150 nm-sized EV formed from intracellular membranes, specifically intraluminal vesicles within endosomes, which are secreted upon fusion with the plasma membrane³⁻⁹; (ii) microvesicles or ectosomes 100-1000 nm-sized EV formed by outward budding from the plasma membrane that contain cytosolic proteins and exhibit common biochemical characteristics, including high levels of phosphatidyl serine and shared surface markers (CD40 ligands, integrins, and selectins)^{10,11}, and (iii) apoptotic bodies, 50-5000 nm-sized EV heterogeneous in size, morphology, and content, as they contain multiple cellular fragments and DNAs^{3,5,10,12}.

Due to the cellular processes associated with EVs biogenesis, different components can be selectively incorporated into each type of EVs. To distinguish EVs derived from the plasma membrane or intracellular membrane compartments, tetraspanins such as CD63, CD81 and CD9 are frequently used¹³⁻¹⁵. CD63 has been suggested to be a key determinator for MVB originating EVs as it is highly retrieved in exosomes along with other tetraspanins¹⁶⁻¹⁹. In recent years, EVs, particularly the exosomes subpopulation, are rising attention in the biomarkers and diagnostic field. All living cells secrete these nanoscopic membrane-derived vesicle, containing produced particles surrounded by a lipid bilayer which transport transmembrane proteins at their surface, as well as other proteins and nucleic acids in their lumen, such as DNAs, mRNAs, miRNAs and other small RNAs³⁻⁵. These vesicles are important mediators of intercellular communication, transferring complex molecular information from one source cell to other cells throughout the body being associated with a variety of physiological functions and pathological disease states^{6,7}.

In particular, exosomes are secreted to the surrounding area of the cells of origin and released to biofluids, such as blood, urine, cerebral spinal fluid, and lymph, thereby reaching distant locations in the body²⁰. Since exosomes derived from many cells in the body can be retrieved in biofluids, tracing their cell of origin, monitoring their cargo and biodistribution and determining their fate in recipient cells remain essential questions still to be addressed in the field^{8,9,21}. A better understanding of EVs content and biomolecule carrier capacity will allow to explore their potential as biomarkers to monitor disease status in response to therapy, particularly in difficult access compartments where biopsies are extremely difficult or not possible to obtain ²²

Liquid biopsies have traditionally been the dominant methodology to study biomarkers. The profile of nucleic acids presents in circulating exosomes isolated from patients in glioblastoma, bladder, liver, colorectal, lung and prostate cancers typically contains double-stranded DNA, tumor-specific mutations and miRNAs dysregulations that allows distinguishing between disease and non-disease conditions²²⁻²⁵. In addition, the protein profile of exosomes has been found dysregulated in several tumors compared with healthy donors^{26,27}.

The fact that exosomes are highly stable in body fluids makes them perfect reservoirs for disease biomarkers and, in particular, tumoral exosomes released into the blood are a great diagnostic source. Cancer cell-derived exosomes spiked into blood sample has been stored for a long period of time making them an excellent tool for biomarker analysis²⁸.

### 1.2. Methods for isolation of exosomes

Despite having several developed protocols in the biomarker field (**Table 1**)^{1,28-30}, isolating exosomes from biofluids is still a challenge due to their heterogeneity, current lack of specific surrogate surface markers for isolation and understanding of their cellular origin¹⁴.

Current purification methods that recover the highest number of extracellular materials are mainly the precipitation polymer kits and lengthy ultracentrifugation (UC) based approach³¹. Such isolation methods cannot distinguish specific exosome populations or separate them from other EV subtypes and free proteins. This limitation hampers the study of biomarkers from tissue-specific exosomes and masks important biosignatures, making unspecified exosomes a major contaminant in preparations^{32,33}. As the perfectly enriched biomarker sources, using exosomes for mapping multi-omic molecular information specific to their pathogenesis in biomarker identification is still extraordinarily challenging.

Polymer-based precipitation, commonly using PEG, is a popular method for isolating vesicles. It creates a hydrophobic environment that causes vesicles to precipitate due to decreased solubility. However, this technique results in low purity due to the coprecipitation of proteins, nucleic acids, and lipoproteins. This contamination hinders the accurate identification of exosome-associated proteins and can lead to false positives or interference in immunoassays and advanced detection platforms^{1,28,31}.

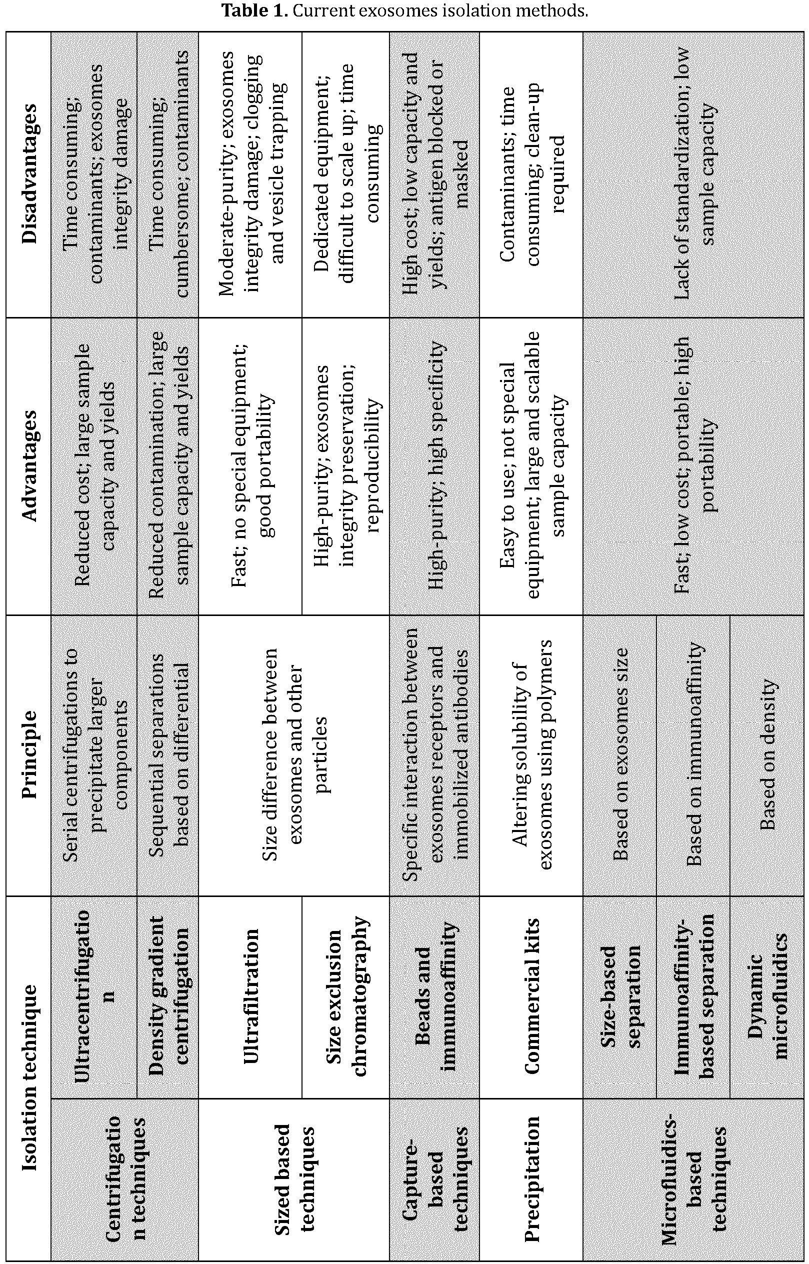

Another approach commonly used is isolation based on immune affinity interactions with exosome surface proteins³⁴⁻³⁸. immunoaffinity isolation is advantageous for recovering exosomes from complex fluids. It provides increased efficiency, specificity, and integrity of captured exosomes. Unlike precipitation-based techniques, immunoaffinity methods offer higher purity and selectivity, reducing costs and hands-on time. These methods target specific surface markers to identify exosome origin and function, including disease markers. Immunomagnetic beads are commonly used in this approach^{36,38}. Exosomes are isolated using magnetic beads with attached antibodies, allowing their separation from other EVs. However, variability in samples and biofluids hampers reproducibility and impacts multi-omic analysis of biomarkers. This is due to issues with antibody specificity, nonspecific adsorption on bead surfaces, high levels of contaminants in biofluids, and inadequate pre-treatment of starting materials.

To counteract these limitations, some nanomaterials were applied in exosome isolation methods, owing to their unique properties and versatile functionalities. For example, nanowires³⁸, metal organic frameworks (MOFs)²¹ and nanostructured graphene oxide (GO)/polydopamine (PDA)³⁹ were used as platforms to increase the surface area to immobilize antibodies and then increase the sensitivity of the assays. Polystyrene beads were coated with Zwitterionic MPC polymers to reduce nonspecific protein binding⁴⁰. The ExoCAS-2 methodology allows the capture and release of exosomes using buffers with different pH, since it is a chitosan-based material that contains polycationic polymer-functionalized magnetic beads that switches by simple adjusting pH⁴¹.

Given the challenges associated with labelling isolated exosomes for characterization and analysis, it is imperative to develop a method that enables labelling of exosomes with specific tags during their isolation from biological fluids. This would address the complexity of handling exosomes separately for labelling and fulfil the actual need for a streamlined approach to facilitate exosome analysis.

To tackle these challenges, the present invention focuses on the development and adapting novel and existing chemistries to enable the efficient isolation of single exosomes derived from tumor cells. This innovative approach will allow to overcome the limitations of current methods.

For that purpose, a bifunctionalized nanodevice has been developed, conjugation a tracking molecule (fluorophore or metal) and a monoclonal antibody for exosome capture. Firstly, the isolation of exosomes using the developed nanodevice was evaluated prior to an optimisation and development of a novel bifunctionalized nanodevice that release the captured cargo for a further analysis. To do so, a novel strategy has been designed to conjugate exosome-specific antibodies targeting CD63, CD81 and CD9 tetraspanins through a covalent approach, maleimide-thiol bond. This innovative method allows for efficient and precise labelling of exosomes, enabling robust characterization and analysis of these small vesicles with improved specificity and accuracy.

Finally, the validation of the novel nanodevice for the selective capture, labelling with fluorescent or metal tags for the consequent and innocuous release of exosomes from biological fluids to further analyzed by flow mass cytometry for cancer diagnosis.

### 1.3 DEVELOPMENT OF A NANODEVICE FOR THE IMMUNE-CAPTURE AND RELEASE OF EXOSOMES BASED ON MONOCLONAL ANTIBODY SPECIFIC RECOGNITION

As previously described, in order to overcome the challenges associated with labelling isolated exosomes for characterization and analysis, it is crucial to develop a method that allows for labeling of exosomes with specific tags during their isolation from biological fluids. This approach would address the complexity of handling exosomes separately for labeling and fulfill the practical need for a streamlined approach to facilitate exosome analysis. By enabling direct labeling of exosomes during the isolation process, this method would offer a more efficient and convenient way to study exosomes, eliminating the need for additional handling steps and minimizing potential artifacts or loss of exosome integrity. Such a streamlined approach would greatly enhance the accuracy and reliability of exosome characterization and analysis, leading to a deeper understanding of their biological functions and potential applications in various fields, including diagnostics, therapeutics, and biomarker discovery.

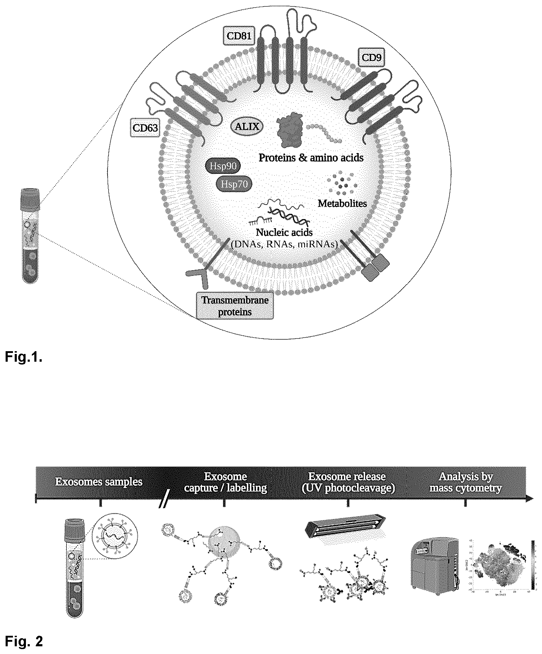

To overcome these exosomes analysis issues, the development of a novel strategy to capture exosomes using nanotechnology was achieved, incorporating a cleavable linker to release the exosomes captured due to antibody specific recognition when it is exposed to UV light. In this case, as a proof of concept, a nanodevice was developed combining a monoclonal antibody against CD63, and a metal (terbium), as tracking molecule, in order to be analyzed by mass cytometry (**Figure 2**). The use of mass cytometry for detecting exosomes would enable the simultaneous labelling of multiple biomarkers and their analysis at the single-cell level. This approach would provide a comprehensive dataset of information from the sample in a single step, offering a valuable tool for studying exosomes. Exosomes were obtained from TNC breast cancer cells.

### Results

### Preparation of nanodevice for immune-capture and release of exosomes

In this case, larger cross-linked aminomethyl nanoparticles were synthesized *via* dispersion polymerization⁴² in an inert atmosphere (argon), using an ethanol/water mixture (86:14) as the dispersion medium. Poly(N-vinylpyrrolidone) (PVP) with a molecular weight of 29,000 g/mol served as the stabilizer, while VBAH was employed as the amino-functionalized monomer and 2,2'-azobisisobutyronitrile (AIBN) as the radical initiator. The reaction was conducted at a controlled temperature of 65°C, stirring at 350 rpm under an inert atmosphere of argon. Once the reaction was complete, the nanoparticle suspension was allowed to cool for 30 minutes, followed by purification through washing with methanol and water, centrifugation, and dispersion cycles (Scheme 1). The resulting nanoparticles were of 580 nm of diameter.

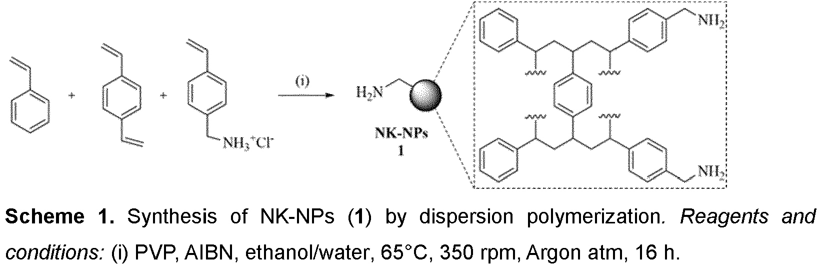

To prepare the cleavable nanodevice designed for immune-capture and release of exosomes, aminomethyl nanoparticles (NK-NPs (1)) were conjugated with a widely-used UV photocleavable linker, amino-3-2-nitrophenyl propionic acid⁴³⁻⁴⁶. The aromatic photolabile group nitrophenyl would enable selective release of the exosomes. In addition, a tracking molecule, such as a lanthanide, was conjugated to label the exosomes for subsequent analysis. Finally, an antibody targeting an exosomal biomarker was conjugated to the nanoparticles to enable specific capture of exosome (**Scheme**). Briefly, NK-NPs (**1**) were conjugated with the photocleavable spacer, prior to a capping process was achieved by incubating the conjugated nanoparticles with a solution of acetic anhydride/N,N-diidopropylethylamine in DMF, obtaining ANPPA-NPs (**26**)**.** To ensure that subsequent conjugations take place exclusively on the photocleavable spacer -NH₂ groups, the capping step is performed to remove any remaining free -NH₂ groups present on the surface of the nanoparticles. Fmoc-Lys(Dde)OH was conjugated to the nanoparticles and after another Fmoc deprotection, Fmoc-PEGs spacer was conjugated. To introduce a thiol functionality on the nanoparticle to be reactive with the maleimide functionalized antibody, 3-[(2-hydroxyethyl)dithio] propanoic acid was conjugated.

Labelling of nanoparticles was carried out by incubating the nanoparticles with DOTA tris(acid)-amido-dPEG₄^{™} TFP ester and N,N-diidopropylethylamine. The nanoparticles were washed and then incubated with a solution of terbium(III) chloride hexahydrate (1 eq), obtaining Tb-NPs (**29**)**.**

Finally, antibody conjugation was achieved by treating the labeled nanoparticles with a solution of dithiothreitol to reduce the thiol group of the conjugated 3-[(2-hydroxyethyl)dithio]propanoic acid. Parallelly, the antibody against CD63 was functionalized with sulfo-SMCC 50 mM and conjugated to NPs to obtain CD63-Tb-NPs (**30**)**.**

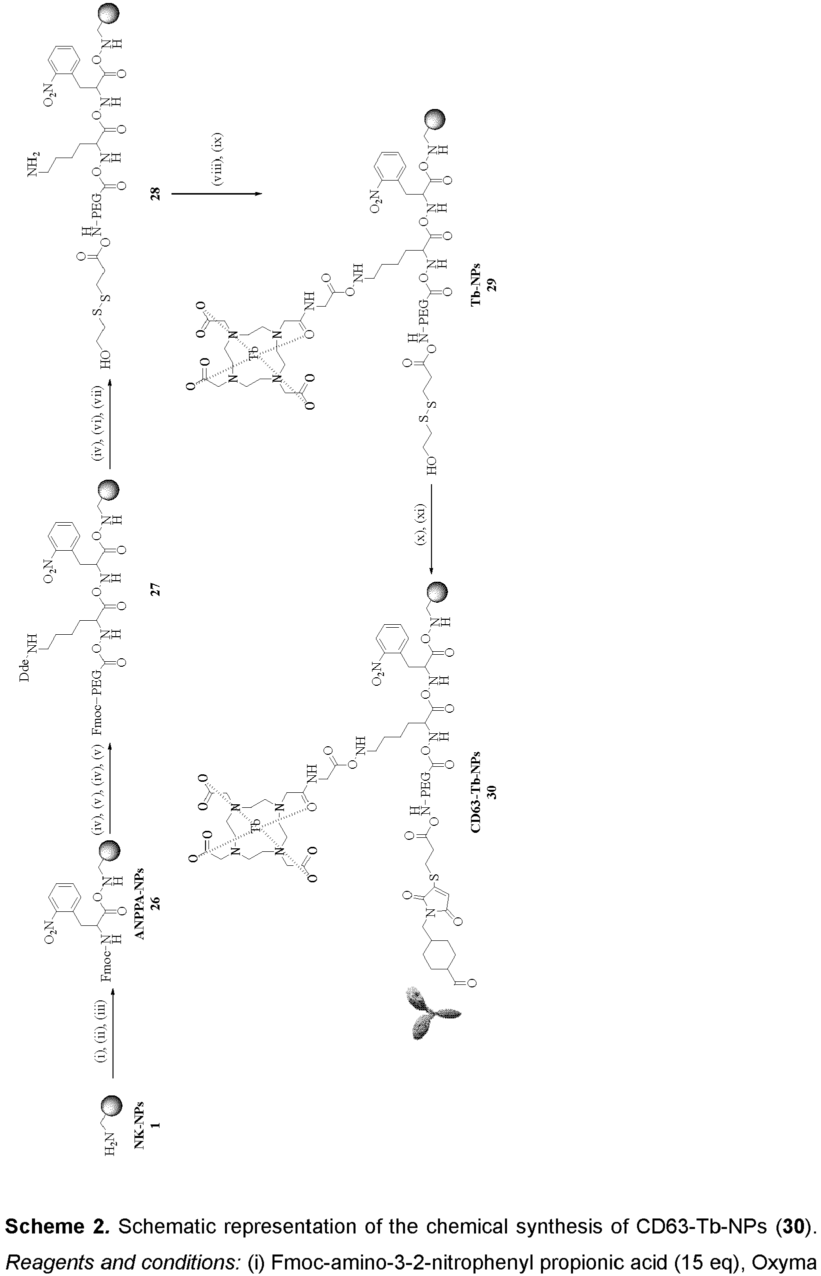

(15 eq), DIC (15 eq), DMF, 2 h, 60° C; (ii) acetic anhydride (50 eq), DIPEA (50 eq), DMF, 30 min, 25°C; (iii) 20% piperidine/DMF, 3 × 20 min; (iv) Fmoc-Lys-Dde (OH) (15 eq), Oxyma (15 eq), DIC (15 eq), DMF, 2 h, 60°C; (v) Fmoc-PEG (15 eq), Oxyma (15 eq), DIC (15 eq), DMF, 2 h, 60°C; (vi) 3-[(2-hydroxyethyl)dithio]propanoic acid (10 eq), Oxyma (10 eq), DIC (10 eq), 2 h, 60°C; (vii) 2% hydrazine/DMF, 3 × 7 min, 25°C; (viii) DOTA tris(acid)-amido-dPEG4 TFP ester (3 eq), DIPEA (3 eq), 15 h, 25°C; (ix) TbCl₃·6H₂O (1 eq), TEA 45 mM, 15 h, 25°C or 50°C; (x) DTT 1 M, H₂O, 3 h, 25°C; (xi) CD-SMCC, PBS, 15h.

### Physical-chemical characterization of developed nanodevices

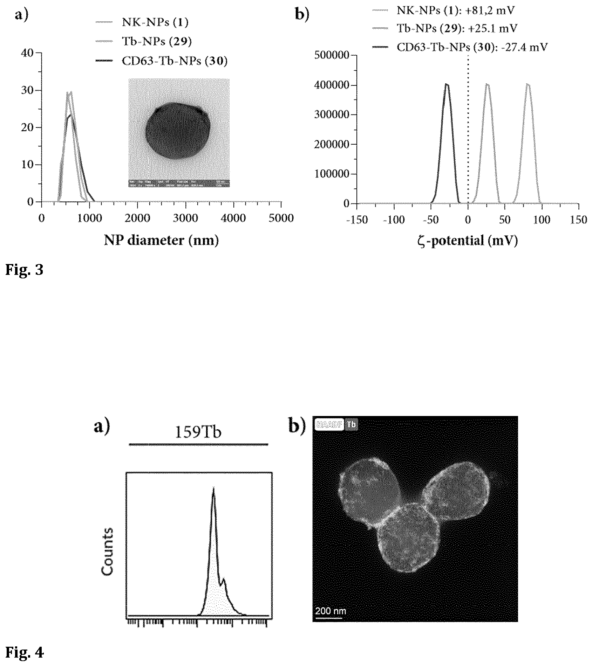

The developed nanodevices for EVs immune-capture and release underwent a thorough physical-chemical characterization. Their size distribution was determined using Dynamic Light Scattering (DLS), which revealed a homogeneous size of 583.2 nm for CD63-Tb-NPs (**30**), and a low polydispersity (PDI of 0.095), as shown in **Figure 3****.** Further evidence of the size of different nanodevices was provided by HRTEM, which showed no aggregation (insets in **Figure 3**). The ζ-potential value for CD63-Tb-NPs (30) was -27.4 mV (**Figure 3**), and it was a more negative value than non-conjugated nanoparticles (NK-NPs (1)), demonstrating the successful conjugation of the different molecules.

The successful conjugation of the lanthanide was confirmed by mass cytometry (**Figure 4a****)** and HRTEM-EDX analysis (**Figure 4b**).

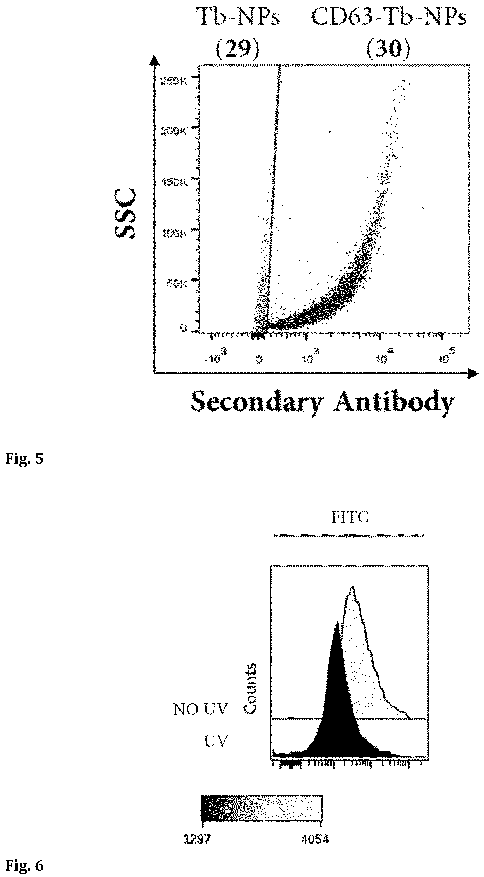

The effectiveness of antibody conjugation was assessed by immunofluorescence (**Figure 5****).** CD63-Tb-NPs (**30**) was tested, while the nanoparticles with no antibody conjugated were used as a negative control. The qualitative analysis of the results confirmed the presence of the antibody conjugated to the NPs.

The efficiency of conjugation and loading capacity (LC) of the nanoparticles with antibodies were estimated and detailed in **Table 2,** with detailed calculations provided in **Section 6.2.3.12.**

**Table 2. Characterization of NPs**

| **Nanoparticles** | **Antibody loading** | |
|---|---|---|
| | **C.E. (%)** | **L.C. (Molec./NP)** |
| **CD63-Tb-NPs (30)** | 100 | 9,50E+03 |

| | | |
|---|---|---|
| **C.E**.= Conjugation efficiency; **L.C**.= Loading capacity; **Molec.**= Molecules | | |

### Evaluation of the functionality of the developed nanodevices

Before assessing the efficacy of the newly developed nanodevices in capturing and releasing exosomes, it was necessary to optimize the protocol for the release process. To evaluate the release efficacy, CD63-Tb-NPs (**30**) were incubated with a fluorescent labelled secondary antibody for 30 minutes, and then exposed to UV light for 5 minutes. After undergoing appropriate washing steps, the resulting nanoparticles were analyzed using flow cytometry. As observed in **Figure 6****,** there is a significant release of nanoparticles' cargo.

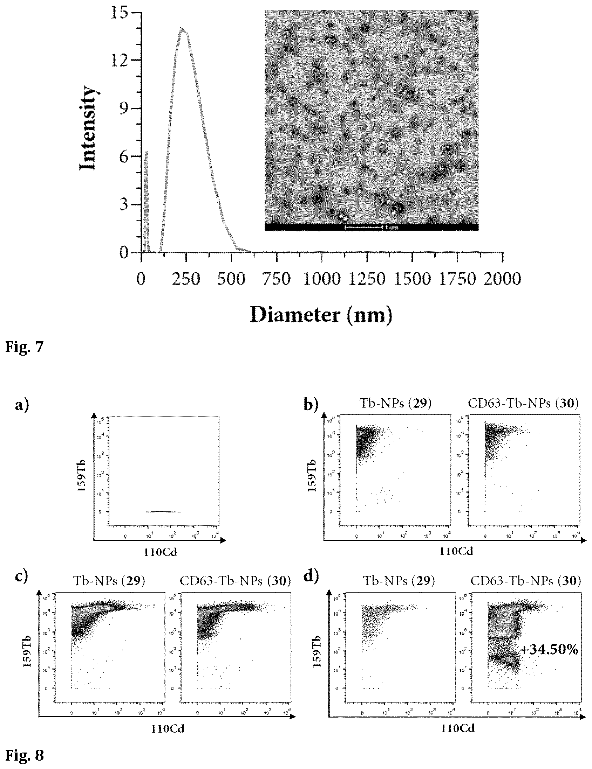

Regarding to exosomes samples, in this case they were obtained from cell culture of TNC breast cancer cell line MDA-MB-468. Size and integrity of exosomes was analyzed by NTA and HRTEM, obtaining most of particles' population with a size around 250 nm **(****Figure 7****).** The isolated exosomes presented a regular morphology after ultracentrifugation steps, and their size were corroborated by HRTEM, after being negatively stained with uranyl acetate to visualize them (inset in **Figure 7**Error! Reference source not found.).

To quantify the isolated exosomes, samples were analyzed by NTA and BCA protein assay. In **Table 3,** concentration of particles per mL and concentration of protein are detailed.

**Table 3. Quantification of exosomes**

| **Technique** | **Quantification** |
|---|---|
| **NTA** | 8,19 x10⁸ particles/mL |
| **BCA Protein Assay** | 0.88 mg/mL |

| | |
|---|---|
| **NTA** = Nanoparticle Tracking Analysis; **BCA** = Bicinchoninic acid assay | |

The capture and release efficiency of exosomes by each nanodevice was evaluated using two methods: (1) mass cytometry, and (2) HRTEM-EDX.

In the first method, exosomes were tagged with a secondary metal-tag antibody. Specifically, an antibody against CD63 was selected and labeled with ¹¹⁰Cd using the Maxpar MCP9 Antibody Labeling Kit from Standard Biotools^{™} (**Figure 8**). CD63-Tb-NPs (**30**) were then incubated with exosomes to assess the capture and release efficiency of these nanodevices, while Tb-NPs (**29**) were used as a negative control. After 30 minutes of incubation at room temperature, samples were stained with ¹¹⁰Cd-antiCD63 (1:100) for 30 min, and then, the experiments were divided into two sets: one set involved exposing half of the CD63-Tb-NPs (**30**) to UV light to facilitate the release of exosomes, while the other half was not exposed to UV light. The samples were analyzed using mass cytometry (**Figure 8**Error! Reference source not found.**a-d**). Despite showing a similar ¹¹⁰Cd signal in the lanthanide positive population (which corresponds to the tracking molecule of the nanodevice) (**Figure 8c**), there was a ¹¹⁰Cd+ signal 34.50% higher in the supernatant of the CD63-Tb-NPs (**30**) sample compared to the Tb-NPs (**29**) after UV exposure (**Figure 8d**). This means that despite there is some adsorption of the exosomes on the nanodevice surface, only the exosomes capture by the antibody of the nanodevice are released.

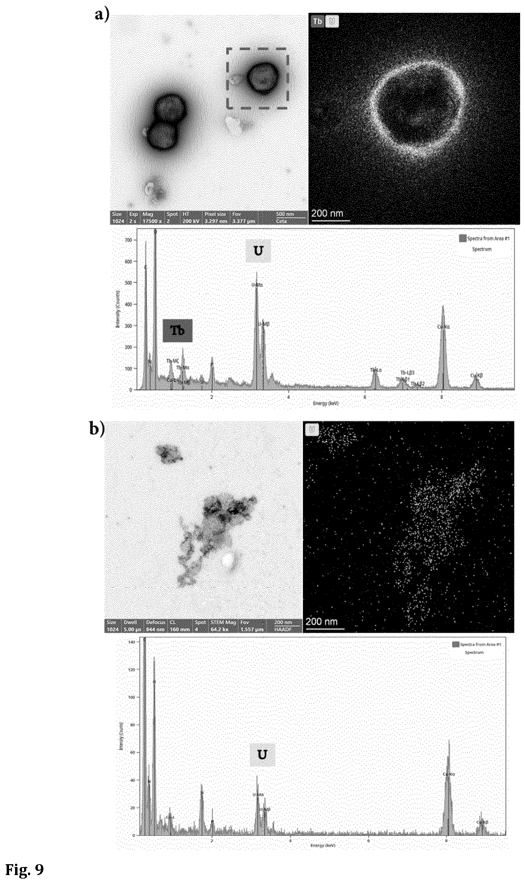

Finally, the exosomes capture and release efficiency of CD63-Tb-NPs (**30**) was also evaluated by HRTEM-EDX analysis. After the incubation of the nanodevice with the exosomes and the UV cleavage, samples were negatively stained with uranyl acetate to visualize exosomes. As shown in **Figure 9****,** exosomes signal was detected by EDX according to the uranium signal. In **Figure 9**Error! Reference source not found.**a** (samples with CD63-Tb-NPs (**30**) prior to UV cleavage), small low-electronic enriched objects could be seen all over the nanodevices surfaces. Further analysis by EDX technique showed that these small particles had high uranium signal, which corresponds to exosomes. In **Figure 9b****,** the supernatant with the cargo release after exosomes capture was analyzed, and exosomes could be found in the sample.

In summary, we have successfully optimized the previously developed nanodevice for immune-capture of extracellular vesicles (EVs). This innovative nanodevice enables the labeling and controlled release of isolated exosomes, which can be tracked using mass cytometry. By utilizing this technique, we can obtain a comprehensive proteomic profile of the exosomes. The release mechanism of the exosomes is facilitated by the conjugation of a UV photolabile linker. We have demonstrated the functionality of this nanodevice by effectively isolating exosomes from a TNC cell line. To establish its versatility, further development is required, including targeting other exosomal biomarkers.

### References

1. Wang, W., Luo, J. & Wang, S. Recent Progress in Isolation and Detection of Extracellular Vesicles for Cancer Diagnostics. Adv Healthc Mater 7, 1800484 (2018).
2. Lemery, S., Keegan, P. & Pazdur, R. First FDA Approval Agnostic of Cancer Site - When a Biomarker Defines the Indication. N Engl J Med 377, 1409-1412 (2017).
3. Raposo, G. & Stoorvogel, W. Extracellular vesicles: exosomes, microvesicles, and friends. J Cell Biol 200, 373-383 (2013).
4. Rufino-Ramos, D. et al. Extracellular vesicles: Novel promising delivery systems for therapy of brain diseases. J Control Release 262, 247-258 (2017).
5. Van Niel, G., D'Angelo, G. & Raposo, G. Shedding light on the cell biology of extracellular vesicles. Nature Reviews Molecular Cell Biology 2018 19:4 19, 213-228 (2018).
6. Abels, E. R. & Breakefield, X. O. Introduction to Extracellular Vesicles: Biogenesis, RNA Cargo Selection, Content, Release, and Uptake. Cell Mol Neurobiol 36, 301-312 (2016).
7. Stahl, P. D. & Raposo, G. Extracellular Vesicles: Exosomes and Microvesicles, Integrators of Homeostasis. Physiology (Bethesda) 34, 169-177 (2019).
8. Kalluri, R. & LeBleu, V. S. The biology, function, and biomedical applications of exosomes. Science (1979) 367, (2020).
9. Tan, Y. et al. Tumor-derived exosomal components: the multifaceted roles and mechanisms in breast cancer metastasis. Cell Death & Disease 2021 12:6 12, 1-18 (2021).
10. Maas, S. L. N., Breakefield, X. O. & Weaver, A. M. Extracellular Vesicles: Unique Intercellular Delivery Vehicles. Trends Cell Biol 27, 172-188 (2017).
11. Mathieu, M., Martin-Jaular, L., Lavieu, G. & Théry, C. Specificities of secretion and uptake of exosomes and other extracellular vesicles for cell-to-cell communication. Nat Cell Biol 21, 9-17 (2019).
12. van Niel, G. et al. Challenges and directions in studying cell-cell communication by extracellular vesicles. Nat Rev Mol Cell Biol 23, 369-382 (2022).
13. Andreu, Z. & Yáñez-Mó, M. Tetraspanins in extracellular vesicle formation and function. *Front Immunol* **5,** 442 (2014).
14. Théry, C. et al. Minimal information for studies of extracellular vesicles 2018 (MISEV2018): a position statement of the International Society for Extracellular Vesicles and update of the MISEV2014 guidelines. https://doi.org/10.1080/20013078.2018.1535750 7, (2018).
15. Liu, Y. et al. Extracellular vesicle tetraspanin-8 level predicts distant metastasis in non-small cell lung cancer after concurrent chemoradiation. Sci Adv 6, 6162-6173 (2020).
16. Mathieu, M. et al. Specificities of exosome versus small ectosome secretion revealed by live intracellular tracking of CD63 and CD9. Nature Communications 2021 12:1 12, 1-18 (2021).
17. Corso, G. et al. Systematic characterization of extracellular vesicle sorting domains and quantification at the single molecule - single vesicle level by fluorescence correlation spectroscopy and single particle imaging. J Extracell Vesicles 8, 1663043 (2019).
18. Hurwitz, S. N. et al. CD63 Regulates Epstein-Barr Virus LMP1 Exosomal Packaging, Enhancement of Vesicle Production, and Noncanonical NF-κB Signaling. J Virol 91, (2017).
19. Corrigan, L. et al. BMP-regulated exosomes from Drosophila male reproductive glands reprogram female behavior. Journal of Cell Biology 206, 671-688 (2014).
20. Ricklefs, F. L. et al. Immune evasion mediated by PD-L1 on glioblastoma-derived extracellular vesicles. Sci Adv 4, (2018).
21. Zhang, H., Zhang, Q., Deng, Y., Chen, M. & Yang, C. Improving Isolation of Extracellular Vesicles by Utilizing Nanomaterials. Membranes 2022, Vol. 12, Page 55 12, 55 (2021).
22. Skog, J. et al. Glioblastoma microvesicles transport RNA and proteins that promote tumour growth and provide diagnostic biomarkers. Nat Cell Biol 10, 1470-1476 (2008).
23. Fais, S. et al. Evidence-Based Clinical Use of Nanoscale Extracellular Vesicles in Nanomedicine. ACS Nano 10, 3886-3899 (2016).
24. Balaj, L. et al. Tumour microvesicles contain retrotransposon elements and amplified oncogene sequences. Nature Communications 2011 2:1 2, 1-9 (2011).
25. Corcoran, C., Rani, S. & O'Driscoll, L. miR-34a is an intracellular and exosomal predictive biomarker for response to docetaxel with clinical relevance to prostate cancer progression. Prostate 74, 1320-1334 (2014).
26. Peinado, H. et al. Melanoma exosomes educate bone marrow progenitor cells toward a pro-metastatic phenotype through MET. Nature Medicine 2012 18:6 18, 883-891 (2012).
27. Melo, S. A. et al. Glypican-1 identifies cancer exosomes and detects early pancreatic cancer. Nature 2015 523:7559 523, 177-182 (2015).
28. Kalra, H. et al. Comparative proteomics evaluation of plasma exosome isolation techniques and assessment of the stability of exosomes in normal human blood plasma. Proteomics 13, 3354-3364 (2013).
29. Li, P., Kaslan, M., Lee, S. H., Yao, J. & Gao, Z. Progress in Exosome Isolation Techniques. Theranostics 7, 789 (2017).
30. Zhu, L. et al. Isolation and characterization of exosomes for cancer research. Journal of Hematology & Oncology 2020 13:1 13, 1-24 (2020).
31. Niu, Z. et al. Polymer-based precipitation preserves biological activities of extracellular vesicles from an endometrial cell line. PLoS One 12, e0186534 (2017).
32. Liu, C. et al. Single-Exosome-Counting Immunoassays for Cancer Diagnostics. Nano Lett 18, 4226-4232 (2018).
33. Wang, J., Ma, P., Kim, D. H., Liu, B. F. & Demirci, U. Towards microfluidic-based exosome isolation and detection for tumor therapy. Nano Today 37, 101066 (2021).
34. Popovic, M., Mazzega, E., Toffoletto, B. & de Marco, A. Isolation of anti-extracellular vesicle single-domain antibodies by direct panning on vesicle-enriched fractions. Microb Cell Fact 17, 6 (2018).
35. Poellmann, M. J. et al. Immunoavidity-Based Capture of Tumor Exosomes Using Poly(amidoamine) Dendrimer Surfaces. Nano Lett 20, 5686-5692 (2020).
36. Campos-Silva, C. et al. High sensitivity detection of extracellular vesicles immune-captured from urine by conventional flow cytometry. Scientific Reports 2019 9:1 9, 1-12 (2019).
37. Islam, M. K. et al. A Nanoparticle-Based Approach for the Detection of Extracellular Vesicles. Scientific Reports 2019 9:1 9, 1-9 (2019).
38. Lim, J. et al. Direct isolation and characterization of circulating exosomes from biological samples using magnetic nanowires. J Nanobiotechnology 17, 1-12 (2019).
39. Zhang, P., He, M. & Zeng, Y. Ultrasensitive microfluidic analysis of circulating exosomes using a nanostructured graphene oxide/polydopamine coating. Lab Chip 16, 3033-3042 (2016).
40. Yoshida, M. et al. Preferential capture of EpCAM-expressing extracellular vesicles on solid surfaces coated with an aptamer-conjugated zwitterionic polymer. Biotechnol Bioeng 115, 536-544 (2018).
41. Kim, H. & Shin, S. ExoCAS-2: Rapid and Pure Isolation of Exosomes by Anionic Exchange Using Magnetic Beads. Biomedicines 2021, Vol. 9, Page 28 9, 28 (2021).
42. Unciti-Broceta, A., Johansson, E. M. V., Yusop, R. M., Sánchez-Martín, R. M. & Bradley, M. Synthesis of polystyrene microspheres and functionalization with pd0 nanoparticles to perform bioorthogonal organometallic chemistry in living cells. Nat Protoc 7, 1207-1218 (2012).
43. Xue, Y. et al. Stimulus-cleavable chemistry in the field of controlled drug delivery. Chem Soc Rev 50, 4872-4931 (2021).
44. Brown, B. B., Wagner, D. S. & Geysen, H. M. A single-bead decode strategy using electrospray ionization mass spectrometry and a new photolabile linker: 3-amino-3-(2-nitrophenyl)propionic acid. Mol Divers 1, 4-12 (1995).
45. Bosques, C. J. & Imperiali, B. Photolytic control of peptide self-assembly. J Am Chem Soc 125, 7530-7531 (2003).
46. Ariyasu, S. et al. Selective capture and collection of live target cells using a photoreactive silicon wafer device modified with antibodies via a photocleavable linker. Langmuir 28, 13118-13126 (2012).
47. Whiteside, T. L. Tumor-Derived Exosomes and Their Role in Cancer Progression. Adv Clin Chem 74, 103-141 (2016).
48. Costa-Silva, B. et al. Pancreatic cancer exosomes initiate pre-metastatic niche formation in the liver. Nature Cell Biology 2014 17:6 17, 816-826 (2015).

## Claims

1. A nanoparticle conjugate of formula A-X-B-C, wherein:
a. A is a nanoparticle preferably an amino nanoparticle such as an amino polystyrene nanoparticle (as an amino methyl nanoparticle),
b. X is a linker that is cleaved when exposed to an ultraviolet wavelength;
c. B is one or more amino acids or analogues thereof, preferably a lysine having the N-α-amino and N-ε groups thereof optionally protected by orthogonal protecting groups such as Dde and Fmoc, preferably Fmoc-Lys (Dde); and
d. C is a bioactive molecule such as an antibody that binds specifically to a target molecule, in particular to an extracellular vesicle (preferably an exosome),
wherein each of A and X, X and B, and B and C are directly linked to each other, optionally through a linker.

2. The nanoparticle conjugate of claim 1, wherein the nanoparticle (A) is capable of being functionalized with (a) at least one imaging agent (T), and at least one bioactive molecule (D), and said conjugate is **characterized by**
a. the nanoparticle A being directly linked, preferably via an amide bond, optionally through a linker, to the cleavable spacer;
b. the cleavable spacer (X) being further directly linked, preferably via an amide bond, optionally through a linker such as PEG, to one or more amino acids or analogues thereof (B); and
c. the one or more amino acids or analogues thereof (B) being further directly linked, preferably via an amide bond, optionally through a linker such as PEG, to a bioactive molecule or, also optionally through the linker such as PEG, to a suitable molecule capable of binding a bioactive molecule.

3. The nanoparticle conjugate of any of claims 1 or 2, wherein the the cleavable spacer is amino-3-2-nitrophenyl propionic acid.

4. The nanoparticle conjugate of any of claims 1 to 3, wherein the suitable molecule capable of binding a bioactive molecule is such as 3-[(2-hydroxyethyl)dithio]propanoic acid.

5. The nanoparticle conjugate of any of claims 1 to 4, wherein the bioactive molecule is an extracellular vesicle (preferably exosome) binding agent such as an antibody or fragment thereof (such as antiCD63, antiCD81 and/or antiCD9 antibody or fragment thereof).

6. The nanoparticle conjugate of any of claims 1 to 5, wherein the nanoparticle conjugate is functionalized with (a) at least one imaging agent (T), wherein the T is bonded to the N-α-amino and/or N-ε groups once deprotected.

7. The nanoparticle conjugate of claim 6, wherein the imaging agent (T) is a fluorophore, preferably a far-red cyanine derivative (Cy7) or a metal such as a chloride hexahydrate lanthanide salt.

8. A method for producing nanoparticles (NP) that can be bi-functionalised, comprising the following steps:
a. conjugating the NPs to a cleavable spacer to provide cleavable NPs;
b. conjugating the resultant cleavable NPs of step a) to one or more amino acids or analogues thereof **characterized by** comprising orthogonal protecting groups such as Dde and Fmoc, wherein the conjugation step b) is optionally followed or preceded by one or more PEGylation steps; and
c. conjugating the product resultant from step b), optionally followed or preceded by one or more PEGylation steps, with a chemical group capable of binding to a binding agent such as an extracellular vesicle binding agent, such as an antibody.

9. The method of claim 8, wherein the nanoparticles are polystyrene nanoparticles (NPs), or amino NPs or preferably polystyrene amino NPs.

10. The method of anyone of claims 8 to 9, wherein the the cleavable spacer is amino-3-2-nitrophenyl propionic acid.

11. The method of anyone of claims 8 to 10, wherein the suitable molecule capable of binding a bioactive molecule is such as 3-[(2-hydroxyethyl)dithio]propanoic acid.

12. The method of anyone of claims 8 to 11, wherein the bioactive molecule is an extracellular vesicle (preferably exosome) binding agent such as an antibody or fragment thereof (such as antiCD63, antiCD81 and/or antiCD9 antibody or fragment thereof).

13. A method of producing functionalised nanoparticles (NP), wherein the method comprises starting with the product of the method of any one of claims 8 to 12:
a. removing the orthogonal protecting group, such as Dde, for conjugation of the nanoparticle to a tracking molecule such as a fluorophore or a metal such as a chloride hexahydrate lanthanide salt; and/or
b. further conjugating the product of step a) to a binding agent such as an extracellular vesicle binding agent, such as an antibody.

14. A nanodevice comprising the nanoparticle conjugate of any one of claims 1 to 7.

15. In vitro use of the nanodevice of claim 14 for isolating extracellular vesicles, preferably exosomes.
